# EUROPEAN PATENT APPLICATION

(11) **EP 3 903 700 A1**
(43) Date of publication of application: **03.11.2021**
(21) Application number: 19905793.6
(22) Date of filing: 20.12.2019
(51) Int. Cl.: A61B 17/32

(54) **ULTRASONIC SURGICAL INSTRUMENT**

(30) Priority: 27.12.2018 CN 201811613552
(71) Applicant: Ezisurg Medical Co., Ltd., Shanghai 201201 (CN); Ezisurg (Suzhou) Medical Co., Ltd., Suzhou, Jiangsu 215163 (CN)
(72) Inventor: NIE, Honglin, Shanghai 201318 (CN); CHEN, Jidong, Shanghai 201318 (CN); LI, Zhidong, Shanghai 201318 (CN); ZHU, Guozheng, Shanghai 201318 (CN); CHANG, Wangtao, Shanghai 201318 (CN); ZHANG, Guangbin, Shanghai 201318 (CN); GU, Xiaoye, Shanghai 201318 (CN)
(74) Representative: Mansfield, Peter Turquand
(86) International application number: PCT/CN2019/126974
(87) International publication number: WO 2020/135254

(57) **Abstract**

An ultrasonic surgical instrument (3) having a side-button triggered coagulation function, including a cutting button (7), a coagulation button (5), a scalpel bar (31), a clamp (32), a cannula assembly (33), a handle (6), and an internal structure thereof, wherein the cutting button (7) is pressed by an index finger to trigger a cutting function, and the coagulation button (5) is pressed by a thumb to trigger the coagulation function. The ultrasonic surgical instrument can reduce misoperation of a surgeon during actual surgery, and relieves fatigue of the surgeon. Moreover, the side-button triggered coagulation function can be triggered by a left or a right hand of the surgeon, and the surgeon who can operate with both the left hand and the right hand can operate the ultrasonic surgical instrument conveniently.

## Description

### TECHNICAL FIELD

The present invention relates to an ultrasonic surgical instrument, and more particularly to an ultrasonic surgical instrument having a side-button triggered coagulation function.

### BACKGROUND

With the popularization of minimally invasive surgeries, ultrasonic scalpels have become conventional surgical instruments. The ultrasonic scalpel enables a scalpel tip to oscillate mechanically at a certain ultrasonic frequency by means of an ultrasonic frequency generator, such that water molecules in a tissue are vaporized, protein hydrogen bonds are broken, and cells are disintegrated, so as to cut or coagulate the tissue or seal blood vessels. The ultrasonic scalpel can simultaneously complete tissue cutting and coagulation, and has a little lateral thermal damage.

The ultrasonic scalpel primarily consists of an ultrasonic frequency generator, a transducer, and a surgical instrument, wherein the ultrasonic frequency generator generates an oscillating electrical signal; the transducer converts the oscillating electrical signal into mechanical vibration; the surgical instrument utilizes the mechanical vibration of the transducer to cut off and coagulates a tissue. The surgical instrument generally consists of a scalpel bar, a clamp forming a clamping structure together with the scalpel tip (a cutting part at the head of the scalpel bar), a cannula surrounding the scalpel bar, a handle, and a holding mechanism. The scalpel bar transfers the mechanical vibration of the transducer to the scalpel tip; The scalpel tip and clamp cooperate to clamp the tissue to realize cutting and coagulation functions; on one hand, the cannula isolates the scalpel bar from the exterior, and has the effects of protecting and supporting the scalpel bar, and on the other hand, the cannula and the clamp form a link mechanism for driving the clamp to close and open; the handle and the holding mechanism are held in a hand of a surgeon, and can be operated to open and close the clamp; and switch buttons, which are generally called a cutting button and a coagulation button, can control the ultrasonic frequency generator to start to output or stop outputting the oscillating electrical signal. As the name implies, the cutting button mainly completes the function of tissue stripping and cutting, and the coagulation button mainly completes the function of tissue coagulation or vessel sealing. During actual surgery, the frequency used for the cutting function is higher than that for the coagulation function.

At present, the cutting button and the coagulation button of most ultrasonic surgical instruments are both arranged at a position an index finger can press on a front surface of the ultrasonic scalpel, and are both pressed by the index finger to trigger functions; therefore, it would be easy to cause misoperation during use; furthermore, the frequency used for the cutting function is higher than that for the coagulation function during actual surgery; the index finger presses the two buttons in turn in a long time, which may easily cause fatigue, thereby further improving the false triggering risk.

In order to solve the problem of false triggering, the existing ultrasonic surgical instrument provides a plurality of salient detent points on one button, and provides a smooth surface for the other button. During surgery, the surgeon needs to determine the cutting button or the coagulation button by means of the salient points before the triggering, and then performs tissue cutting or coagulation surgery. The design can reduce the false triggering risk to a certain extent, but cannot avoid the fatigue of the index finger caused by the long time in-turn pressing of the two buttons. In addition, the finger would suffer pain after pressing the salient detent points for a long time. In another design solution of the ultrasonic surgical instrument, the cutting button and the coagulation button are both arranged on an oblique lateral surface of the ultrasonic scalpel; the cutting button and the coagulation button are pressed and triggered by a thumb. However, the thumb needs to be raised up higher than a normal position during pressing, which makes the thumb operation more difficult. The coagulation button and the cutting button are both pressed and triggered by a thumb pressing, which also has the false triggering risk. In still another design solution of the ultrasonic surgical instrument in the prior art, in order to avoid confusing the two buttons, one of the buttons is arranged on a lateral surface of the instrument. However, the button is arranged in a vertical direction on the lateral surface, at which position the thumb would be uncomfortable to operate; furthermore, the patent does not disclose a detailed circuit switching solution.

### SUMMARY

In order to solve the above technical problems, reduce false triggering of a surgeon during actual surgery, and relieve fatigue of the surgeon, in one aspect, the present invention provides an ultrasonic surgical instrument, including a scalpel bar, a first button, a second button, a handle, a transmission mechanism, and other internal structures, wherein the first button is located on a front surface of the instrument; the second button is located on an oblique lateral surface of the instrument; and the first button and/or the second button can slide or rotate by a certain distance. Preferably, the first button is a cutting button, and the second button is a coagulation button.

As a further option, the handle includes a left housing, a right housing, and other components.

As a further option, the cutting button is pressed by an index finger to trigger a cutting function, and the coagulation button is pressed by a thumb to trigger a coagulation function.

As a further option, the second button can be symmetrically disposed on oblique surfaces on two sides of the instrument.

As a further option, the second button rotates around a rotary pin; the rotary pin penetrates through a mounting hole of the second button, and the two ends thereof are respectively mounted in rotary pin holes of the left and the right housings.

As a further option, the rotary pin penetrates through the transmission mechanism, so as to connect the second button to the transmission mechanism in parallel.

As a further option, a limit surface of the second button is limited by a housing limit plane of the handle, such that the second button can only rotate around the rotary pin.

As a further option, a circuit board is disposed in the handle, and the first button and the second button trigger functions by pressing the circuit board.

As a further option, the first button and/or the second button can slide a certain distance, thereby avoiding misoperation.

As a further option, the first button is disposed in a mounting groove; the mounting groove consists of a guide chute, an upper guide platform, and a lower guide platform; and a guide boss of the first button slides in the guide chute.

As a further option, an elastic mechanism is disposed between the first button, the second button and the circuit board.

Preferably, the elastic mechanism can be one or more of a leaf spring, an elastic sheet, and a spring which are provided with an elastic structure.

As a further option, the elastic mechanism is fixedly mounted in/on a groove, a gap, a platform or a combined structure of the left and the right housings of the handle.

As a further option, a first trigger metal dome and a second trigger metal dome on the circuit board are respectively mounted on two sides of the circuit board, such that the first button and the second button can be pressed in reverse directions to trigger functions.

As a further option, the circuit board consists of a flexible board and a hard board; the flexible board is connected to an external signal structure assembly of the surgical instrument; and the hard board is fixedly mounted in a circuit board mounting groove of the left and the right housings of the handle.

As a further option, the elastic mechanism has split structures which respectively provide the resilience of the first button and the resilience of the second button.

As a further option, the elastic mechanism has an integrated structure which can simultaneously provide the resilience of the first button and the resilience of the second button.

As a further option, a limit boss is designed for the housings and/or the first button and/or the second button, so as to prevent the structure from being damaged due to the over-pressing of the buttons.

As a further option, a limit boss is designed for the housings and/or the first button and/or the second button, so as to keep a gap between a contact surface and the housing.

In another aspect, the present invention provides a method for operating an ultrasonic surgical instrument. Specifically, a first button located on a front surface of the instrument is pressed and triggered by an index finger, and a second button located on oblique lateral surfaces on two sides of the instrument is pressed and triggered by a thumb.

As a further option, the second button can rotate around a rotation axis by a rotation distance of 1-30 degrees.

With the above structural arrangement, the present invention has the following beneficial effects:
first, a false triggering frequency when the cutting function and the coagulation function are switched with an index finger is reduced;
second, the coagulation function is triggered by a thumb, thereby improving the comfort of the cutting button and relieving the fatigue of the index finger; and
third, the cutting button on the front surface and the coagulation button on the oblique lateral surface can only be triggered after sliding a certain distance, thereby further improving the operation comfort of the surgeon and reducing the false triggering risk.

Moreover, the side-button triggered coagulation function can be triggered by a left or a right hand of the surgeon, and the surgeon who can operate with both the left hand and the right hand can operate the instrument of the present invention conveniently.

### BRIEF DESCRIPTION OF THE DRAWINGS

The drawings are provided to further understand the present application, constitute a part of the specification, and are used, together with specific embodiments of the present application, to explain the present application, but are not intended to limit the present application.
Fig. 1 is a schematic view of an ultrasonic surgical instrument system;
Fig. 2 is a schematic view of an operating structure of the ultrasonic surgical instrument;
Fig. 3 is a structural schematic view of the ultrasonic surgical instrument;
Fig. 4 is a schematic view of the functions of buttons of the ultrasonic surgical instrument;
Fig. 5 is an installation diagram of a cutting button of the ultrasonic surgical instrument;
Fig. 6 is an installation diagram of a coagulation button of the ultrasonic surgical instrument;
Fig. 7 is an installation diagram of a circuit board and a leaf spring of the ultrasonic surgical instrument; and
Fig. 8 is a structural schematic view of the circuit board of the ultrasonic surgical instrument.

### DETAILED DESCRIPTION OF THE EMBODIMENTS

The present application will be further described hereafter with reference to specific embodiments, wherein the drawings are used for illustrative purpose only, denote schematic views only rather than physical views, and thus should not be considered as a limitation to the present patent. In order to describe the specific embodiments of the present application still better, certain components in the drawings may be omitted, enlarged or minified, and do not denote the sizes of a practical product. A person skilled in the art will understand that certain commonly known structures in the drawings and the descriptions thereof may be omitted. On the basis of the specific embodiments of the present application, all the other specific embodiments obtained by a person skilled in the art without involving an inventive effort are included in the protection scope of the present application.

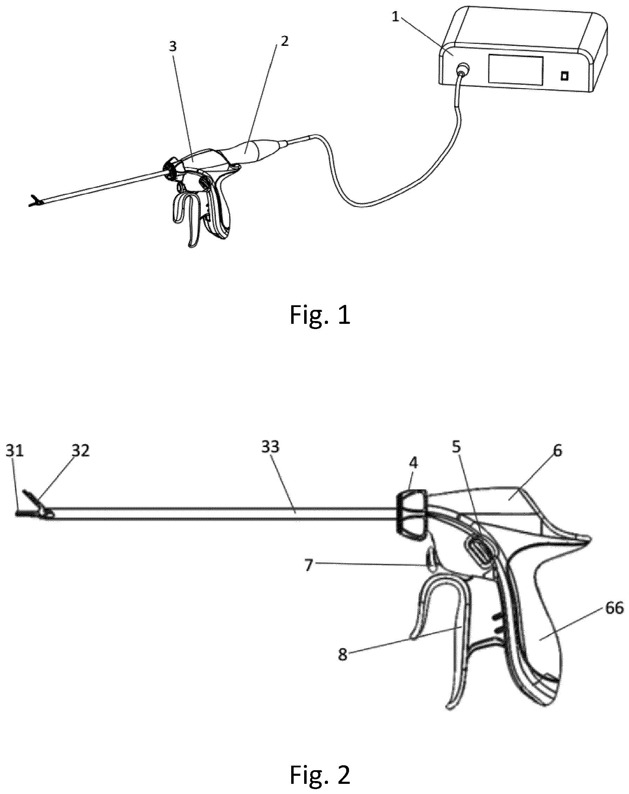

An ultrasonic surgical instrument system, as shown in Fig. 1, mainly consists of an ultrasonic frequency generator 1, a transducer 2, and a surgical instrument 3, wherein the ultrasonic frequency generator 1 generates an oscillating electrical signal; the transducer 2 converts the oscillating electrical signal into mechanical vibration; the surgical instrument 3 utilizes the mechanical vibration of the transducer 2 to cut off and coagulate a tissue.

As shown in Fig. 2, the surgical instrument 3 includes a rotary thumb wheel 4, a coagulation button 5, a handle 6, a cutting button 7, a trigger 8, a scalpel bar 31, a clamp 32, and a cannula assembly 33, wherein the scalpel bar 31 and the clamp 32 forming a clamping structure to perform ultrasonic cutting and coagulation; the cannula assembly 33 is used to isolate from the exterior, protect and support the scalpel bar 31; and the handle 6 includes a transmission system, a trigger resetter, and an electronic device. After the trigger 8 is held tightly, a transmission assembly in the handle 6 drives an inner cannula in the cannula assembly 3 to move, then the clamp 32 and the scalpel bar 31 close; and on the contrary, the clamp opens. The rotary thumb wheel 4 circumferentially rotates, and drives the cannula assembly 33, the scalpel bar 31, and the clamp 32 to rotate, thereby providing convenience for a surgeon to adjust appropriate cutting and coagulation angles.

The coagulation button 5 and the cutting button 7 are arranged as shown Fig. 2; and the coagulation button 5 is located on an oblique lateral surface of the surgical instrument 3. When the surgical instrument is held by a hand, as long as the thumb is slightly raised up and then presses the coagulation button, a coagulation function can be triggered, wherein a movement angle is 0-30 degrees, and preferably 5-10 degrees. The cutting button 7 is located on a front surface of the surgical instrument 3. A cutting function can be triggered by means of pressing with an index finger.

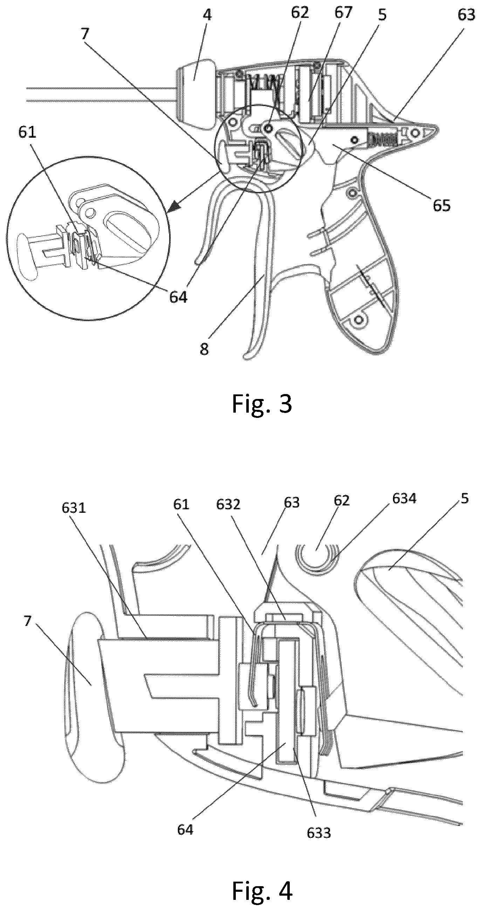

Fig. 3 shows the surgical instrument 3 and internal structures thereof. As shown in the figure, the surgical instrument 3 includes a rotary thumb wheel 4, a coagulation button 5, a cutting button 7, a trigger 8, and a handle 6; internal devices, such as a leaf spring 61, a rotary pin 62, a circuit board 64, a slider 65 and the like, are mounted in a corresponding limit structure of a right housing 63 and a left housing 66 (which are not shown, but can be seen in Fig. 2) of the handle 6. The rotary pin 62 simultaneously penetrates through mounting holes of the coagulation button 5, the trigger 8, and the slider 65; bosses of the right housing 63 and the left housing 66 respectively limit the movement of the button 5, the trigger 8, and the slider 65 without affecting the coagulation button 5; the rotation of the coagulation button 5 does not affect the triggering of the trigger 8 or the sliding of the slider 65 under the drive of the trigger 8, and therefore does not affect the closing of the clamp 32 and the scalpel bar 31. Therefore, owing to the position of the rotary pin 62, the coagulation button 5, the trigger 8, and the slider 65 are connected in parallel, and the rotation and slide do not interfere with each other, thereby reducing the components, enabling the structure to be compact, reducing the structural space, and simplifying the assembly process.

Fig. 4 shows a schematic view of the functions of the buttons of the surgical instrument 3. As shown in the figure, the cutting button 7 and the coagulation button 5 are respectively located on two sides of the circuit board 64; the cutting button 7 is mounted in a cutting button mounting groove 631 of the right housing 63; the leaf spring 61 is mounted in a leaf spring groove 632 of the right housing 63; the circuit board 64 is mounted in a circuit board mounting groove 633 of the right housing 63; the coagulation button 5 is mounted in the right housing 63 by means of the rotary pin 62; the rotary pin 62 is mounted in a pin mounting hole 634 of the right housing 63; when the coagulation button 5 is pressed by a thumb, the coagulation button can rotate around the rotary pin 62; when the cutting button 7 is pressed by an index finger, the cutting button first overcomes an elastic force on the left side of the leaf spring 61, and is then pressed on the circuit board 64 to trigger the cutting function; when the index finger releases the press force, the leaf spring 61 springs the cutting button 7 back; and when the coagulation button 5 is turned, the coagulation button first overcomes an elastic force on the right side of the leaf spring 61, and is then pressed on the circuit board 64 to trigger the coagulation function; when the thumb releases the press force, the leaf spring 61 springs the coagulation button 5 back. It should be noted that the left housing 66 and the right housing 63 of the handle are approximately symmetric structures, and are used to fixedly mount the cutting button 7, the leaf spring 61, the circuit board 64, the coagulation button 5 and the like.

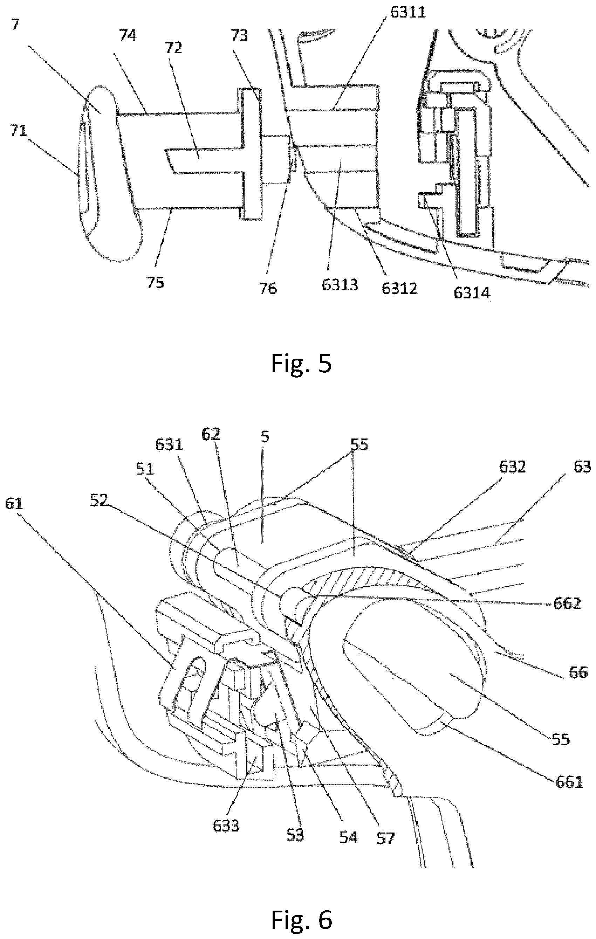

Fig. 5 shows an installation diagram of the cutting button. The cutting button mounting groove 631 in Fig. 4 consists of an upper guide platform 6311, a lower guide platform 6312, and a guide chute 6313 as shown in Fig. 5. When the index finger presses an index finger contact surface 71 of the cutting button 7, a guide boss 72 of the cutting button 7 slides in the guide chute 6313 of the right housing 63, and an upper limit surface 74 and a lower limit surface 75 of the cutting button 7 are respectively limited by the upper guide platform 6311 and the lower guide platform 6312 of the right housing 63, thereby reducing the obliqueness of the cutting button 7. After the cutting button 7 is pressed and slides a certain distance, an elastic sheet contact surface 73 of the cutting button 7 contacts the leaf spring 61, thereby increasing the hand feeling. When a cut triggering boss 76 of the cutting button 7 contacts the circuit board 64, the cutting function is triggered. In order to prevent the circuit board 64 and the mounting fixed structures thereof from being damaged due to the over-pressing of the index finger, a limit stopper 6314 is designed on the right housing 63 to limit the maximum movement distance of the cutting button 7. The left housing 66 of the handle 6 has the corresponding symmetric structure.

Fig. 6 shows an installation diagram of the coagulation button. The rotary pin 62 penetrates through two mounting holes 51 and 52 of the coagulation button 5; two ends of the rotary pin are respectively mounted in a rotary pin hole 634 of the right housing 63 and a rotary pin hole 662 of the left housing; a limit surface 55 of the coagulation button 5 is limited by a limit plane 631 of the right housing 63; correspondingly, the left housing 66 also has a symmetric structure, such that the coagulation button 5 can only rotate around the rotary pin 62. A lower limit boss of the coagulation button 5 and an upper limit boss 632 of the right housing 63 limit the rotation angle of the coagulation button, and ensure that a thumb contact surface 55 of the coagulation button 5 does not touch a limit groove 661 of the left housing 66; and correspondingly, the right housing 63 also has a symmetric structure. When a thumb presses the thumb contact surface 55 of the coagulation button 5, a coagulation triggering boss 53 on the coagulation button 5 rotates, and finally touches the circuit board 64, so as to trigger the coagulation function.

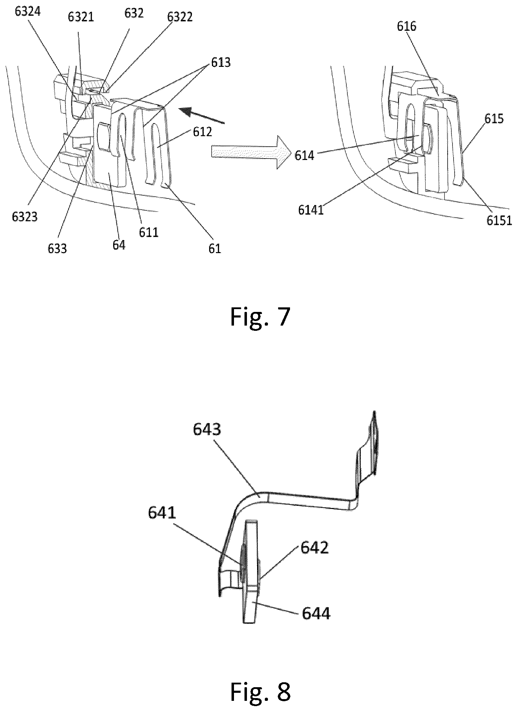

Fig. 7 shows an installation diagram of the circuit board 64 and the leaf spring 61. The circuit board 64 and the leaf spring 61 are sequentially mounted in the corresponding circuit board mounting groove 633 and the leaf spring groove 632 according to a direction as shown by the black arrow in the figure, so as to obtain the right structural schematic view of Fig. 7; the circuit board mounting groove 633 is a rectangular groove having a similar shape to that of a section of the circuit board 64. The leaf spring groove 632 consists of a first upper boss 6321, a second upper boss 6322, and a lower boss 6323; the elastic leaf spring 61 is fixed in an opposite gap by means of staggered arrangement. The leaf spring 61 is inserted in the leaf spring groove 632 until a vertical leaf spring limit platform 613 touches a leaf spring limit platform 6324, so as to limit the positions of a cut triggering boss groove 611 and a coagulation triggering boss groove 612 on the leaf spring 61. The cut triggering boss 76 of the cutting button 7 penetrates through the cut triggering boss groove 611, and the coagulation triggering boss 53 of the coagulation button 5 penetrates through the coagulation triggering boss groove 612. As shown in Fig. 7, the leaf spring 61 can be divided into three parts: a cut spring-back sheet 614, a coagulation spring-back sheet 615, and a fixing sheet 616, wherein the a first convex hull 6141 of the cut spring-back sheet 614 contacts the elastic sheet contact surface 73 of the cutting button 7; a second convex hull 6151 of the coagulation spring-back sheet 615 contacts an elastic sheet contact surface 57 of the coagulation button 5; and the fixing sheet 616 is limited in the leaf spring groove 632.

The structure of the circuit board 64 is as shown in Fig. 8. The circuit board 64 includes a flexible board 643 and a hard board 644; the flexible board 643 is connected to an interface assembly 67 in Fig. 3; the interface assembly 67 in Fig. 3 is connected to the transducer 2 in Fig. 1, and is finally connected to the ultrasonic frequency generator 1 in Fig. 1. When the cutting button 7 and a metal dome 641 or the coagulation button and a metal dome 642 are triggered, the ultrasonic frequency generator controls the mechanical vibration of the scalpel bar 31, so as to perform the cutting and the coagulation functions. In a compact double-sided mounting mode, the metal dome 641 of the cutting button is mounted on a front lateral surface of the hard board 644, and the metal dome 642 of the coagulation button is mounted in a rear lateral surface of the hard board 644; therefore, the mounting space is saved, and the buttons can be triggered in two directions. After the cut triggering boss 76 of the cutting button 7 presses the metal dome 641 of the cutting button by a certain distance, the cutting function is triggered; after the triggering is completed, the cut triggering boss 76 of the cutting button 7 is separated from the metal dome 641 of the cutting button, and the cutting function is deactivated. Similarly, after the coagulation triggering boss 53 of the coagulation button 5 presses the metal dome 642 of the coagulation button by a certain distance, the coagulation function is triggered; after the triggering is completed, the coagulation triggering boss 53 of the coagulation button 5 is separated from the metal dome 642 of the coagulation button, and the coagulation function is deactivated.

It should be noted that the embodiments in the accompanying drawings are merely representative embodiments of the present invention. A person skilled in the art could easily understand that the protection scope of the present invention is not only limited within the scope defined by the embodiments in the accompanying drawings, and the combinations, variations, and changes of the embodiments in the accompanying drawings should all fall in the protection scope of the present invention.

The disclosures above are only the preferred embodiments of the present invention, and are not intended to limit the protection scope of the present invention. Therefore, any equivalent variations made according to the claims of the present invention are all included in the protection scope of the present invention.

## Claims

1. An ultrasonic surgical instrument, **characterized in that** the ultrasonic surgical instrument comprises a scalpel bar, a first button, a second button, a handle, a transmission mechanism, and other internal structures, wherein the first button is located on a front surface of the instrument; the second button is located on an oblique lateral surface of the instrument; the first button and/or the second button can slide or rotate by a certain distance.

2. The ultrasonic surgical instrument according to claim 1, **characterized in that** the first button is a cutting button, and the second button is a coagulation button.

3. The ultrasonic surgical instrument according to claim 1 or 2, **characterized in that** the second button can be symmetrically disposed on oblique surfaces on two sides of the instrument.

4. The ultrasonic surgical instrument according to claim 1 or 2, **characterized in that** the first button and/or the second button rotate/rotates around a rotary pin; the rotary pin penetrates through mounting holes of the first button and/or the second button, and the two ends thereof are respectively mounted in rotary pin holes of left and right housings of the handle.

5. The ultrasonic surgical instrument according to claim 3, **characterized in that** the rotary pin penetrates through the transmission mechanism, so as to connect the second button to the transmission mechanism in parallel.

6. The ultrasonic surgical instrument according to claim 1 or 2, **characterized in that** a limit surface of the second button is limited by a housing limit plane of the handle, such that the second button can only rotate around the rotary pin.

7. The ultrasonic surgical instrument according to claim 1 or 2, **characterized in that** a circuit board is disposed in the handle, and the first button and the second button trigger functions by pressing the circuit board.

8. The ultrasonic surgical instrument according to claim 7, **characterized in that** an elastic mechanism is disposed between the first button, the second button and the circuit board.

9. The ultrasonic surgical instrument according to claim 8, **characterized in that** the elastic mechanism is fixedly mounted in/on a groove, a gap, a platform or a combined structure of left and right housings of the handle.

10. The ultrasonic surgical instrument according to claim 1 or 2, **characterized in that** the first button is disposed in a mounting groove; the mounting groove consists of a guide chute, an upper guide platform, and a lower guide platform; and a guide boss of the first button slides in the guide chute.

11. The ultrasonic surgical instrument according to claim 7, **characterized in that** a first trigger metal dome and a second trigger metal dome are disposed on the circuit board, and are respectively mounted on two sides of the circuit board, such that the first button and the second button can be pressed in reverse directions to trigger functions.

12. The ultrasonic surgical instrument according to claim 7, **characterized in that** the circuit board consists of a flexible board and a hard board; the flexible board is connected to an external signal structure assembly of the surgical instrument; and the hard board is fixedly mounted in a circuit board mounting groove of left and right housings of the handle.

13. The ultrasonic surgical instrument according to claim 8, **characterized in that** the elastic mechanism has split structures which respectively realize the resilience of the first button and the resilience of the second button.

14. The ultrasonic surgical instrument according to claim 8, **characterized in that** the elastic mechanism has an integrated structure which can simultaneously realize the resilience of the first button and the resilience of the second button.

15. The ultrasonic surgical instrument having a side-button triggered coagulation function according to claim 4, **characterized in that** a limit boss is designed for the housings of the handle and/or the first button and/or the second button, so as to prevent the structure from being damaged due to the over-pressing of the buttons.

16. The ultrasonic surgical instrument having a side-button triggered coagulation function according to claim 4, **characterized in that** a limit boss is designed for the housings of the handle and/or the first button and/or the second button, so as to keep a gap between a contact surface and the housing.

17. A method for operating an ultrasonic surgical instrument, **characterized in that** a first button located on a front surface of the instrument is pressed and triggered by an index finger, and a second button located on oblique lateral surfaces on two sides of the instrument is pressed and triggered by a thumb.

18. The method for operating an ultrasonic surgical instrument according to claim 17, **characterized in that** the second button can rotate around a rotation axis by a rotation distance of 1-30 degrees.
